# EUROPEAN PATENT APPLICATION

(11) **EP 4 431 101 A1**
(43) Date of publication of application: **18.09.2024**
(21) Application number: 22892938.6
(22) Date of filing: 09.11.2022
(51) Int. Cl.: A61K 31/7088, A61K 38/21, A61K 45/00, A61K 47/24, A61K 47/69, A61P 1/16, A61P 31/12, A61P 31/20, A61P 37/04, A61P 43/00, C12N 15/117

(54) **THERAPEUTIC AGENT FOR REFRACTORY VIRAL INFECTIONS**

(30) Priority: 10.11.2021 JP 2021183329
(71) Applicant: Tokyo Metropolitan Institute of Medical Science, Tokyo 156-8506 (JP); National University Corporation Kumamoto University, Kumamoto-shi, Kumamoto 860-8555 (JP)
(72) Inventor: KOHARA Michinori, Tokyo 156-8506 (JP); YAMAMOTO Naoki, Tokyo 156-8506 (JP); TANAKA Yasuhito, Kumamoto-shi, Kumamoto 860-8555 (JP); SATO Yusuke, Sapporo-shi, Hokkaido 060-0808 (JP)
(74) Representative: J A Kemp LLP
(86) International application number: PCT/JP2022/042459
(87) International publication number: WO 2023/085440

(57) **Abstract**

The present invention provides a therapeutic agent for refractory viral infections, etc., which is capable of reducing or eliminating cccDNA from hepatocytes. The therapeutic agent for refractory viral infections according to the present invention is characterized by comprising a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.

## Description

### Technical Field

The present invention relates to a therapeutic agent for refractory viral infections, etc.

### Background Art

Hepatitis B virus (HBV) known to cause refractory viral infections is stably maintained in the form of covalently closed circular DNA (cccDNA) structure within the nuclei of hepatic parenchymal cells, thereby resulting in persistent infection (Non-patent Document 1). Thus, the disappearance of cccDNA is required for complete elimination of HBV from the infected cells. Among drugs which have been conventionally used for treatment, reverse transcriptase inhibitors (nucleos(t)ide analogues) (Non-patent Document 2) are incapable of effectively reducing cccDNA, while Peg-IFNα (Non-patent Document 3) is associated with serious adverse events because its target tissue cannot be restricted, and is also incapable of eliminating cccDNA. For this reason, withdrawal from the therapeutic agents has become an important problem, and no techniques have been established to effectively reduce cccDNA.

### Prior Art Documents

Non-patent Document 1: Nassal M. HBV cccDNA: viral persistence reservoir and key obstacle for a cure of chronic hepatitis B. Gut. 2015 Dec; 64 (12): 1972-1984
Non-patent Document 2: De Fraga RS, Van Vaisberg V, Mendes LCA, Carrilho FJ, Ono SK. Adverse events of nucleos(t)ide analogues for chronic hepatitis B: a systematic review. J Gastroenterol 2020; 55: 496-514.
Non-patent Document 3: Craxi A, Antonucci G, Camma C. Treatment options in HBV J Hepatol. 2006; 44 (1 Suppl): S77-83

### Summary of the Invention

Under these circumstances, there has been a demand for the development of a therapeutic agent for refractory viral infections, etc., which is capable of reducing or eliminating cccDNA from hepatocytes (hepatic parenchymal cells).

The present invention has been made in consideration of the above situation, and provides a therapeutic agent for refractory viral infections, etc., as shown below.

(1) A therapeutic agent for refractory viral infections, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.
(2) The therapeutic agent according to (1) above, wherein the molecule imparting the ability to accumulate in hepatocytes is at least one selected from the group consisting of N-acetylgalactosamine, galactose and lactose.
(3) The therapeutic agent according to (1) or (2) above, wherein the drug carrier is at least one selected from the group consisting of lipid nanoparticles, cationic liposomes, atelocollagen and polymeric micelles.
(4) The therapeutic agent according to any one of (1) to (3) above, wherein the poly-I, poly-I analog, poly-C and poly-C analog each independently have a chain length in the range of 50 to 1000 bases.
(5) The therapeutic agent according to any one of (1) to (4) above, wherein the refractory virus is a hepatitis virus.
(6) The therapeutic agent according to (5) above, wherein the hepatitis virus is hepatitis B virus.
(7) The therapeutic agent according to any one of (1) to (6) above, which further comprises a pharmaceutically acceptable additional therapeutic agent for viral infections or is used in combination with the additional therapeutic agent for viral infections, wherein the additional therapeutic agent for viral infections is preferably a nucleos(t)ide analogue.
(8) A pharmaceutical composition for treating refractory viral infections, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.
(9) The pharmaceutical composition according to (8) above, which further comprises a pharmaceutically acceptable additional therapeutic agent for viral infections or is used in combination with the additional therapeutic agent for viral infections, wherein the additional therapeutic agent for viral infections is preferably a nucleos(t)ide analogue.
(10) A method for treating refractory viral infections, which comprises administering the therapeutic agent according to any one of (1) to (7) above or the pharmaceutical composition according to (8) or (9) above to patients with refractory viral infections.
(11) An IFNλ inducer, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.
(12) The inducer according to (11) above, which induces IFNλ in hepatocytes.
(13) A method for inducing IPNλ, which comprises the step of allowing the inducer according to (11) or (12) above to act on hepatocytes.
(14) An IRF1 expression enhancer, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.
(15) The enhancer according to (14) above, which enhances IRF1 expression in hepatocytes.
(16) A method for enhancing IRF1 expression, which comprises the step of allowing the enhancer according to (14) or (15) above to act on hepatocytes.
(17) A Dock11 expression inhibitor, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.
(18) The inhibitor according to (17) above, which inhibits Dock11 expression in hepatocytes.
(19) A method for inhibiting Dock11 expression, which comprises the step of allowing the inhibitor according to (17) or (18) above to act on hepatocytes.
(20) A therapeutic agent for refractory viral infections, which comprises IFNλ and/or IRF 1.
(21) A pharmaceutical composition for treating refractory viral infections, which comprises IFNλ and/or IRF1.
(22) An agent for reducing refractory viral DNA or cccDNA levels, which comprises IFNλ and/or IRF1.
(23) A method for reducing refractory viral DNA or cccDNA levels, which comprises allowing IFNλ and/or IRF1 to act on hepatocytes or allowing IFNλ and/or IRF1 to be expressed in hepatocytes.

### Effects of the Invention

According to the present invention, it is possible to provide a therapeutic agent for refractory viral infections, particularly hepatitis virus infections (especially hepatitis B virus infection), a pharmaceutical composition for treating the same, and a method for treating the same. The therapeutic agent and others are extremely useful because they can be aimed at complete cure of refractory viral infections in terms of being capable of reducing or eliminating viral cccDNA from hepatocytes (hepatic parenchymal cells). Further, according to the present invention, it is also possible to provide an IFNλ inducer, an IRF1 expression enhancer, a Dock11 expression inhibitor and so on, which can be expected to reduce or eliminate the cccDNA. Moreover, according to the present invention, it is also possible to provide an agent for reducing refractory viral DNA or cccDNA levels and a method for reducing refractory viral DNA or cccDNA levels.

### Brief Description of the Drawings

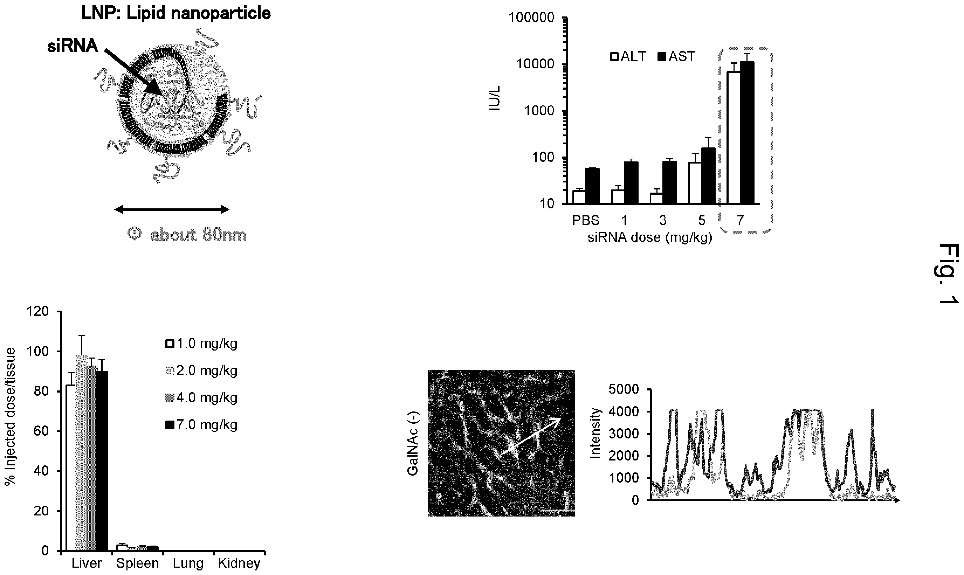

Figure 1 shows the properties of conventional hepatic parenchymal cell-directed pH-responsive lipid nanoparticles.

400 µL of a 90% tert-butanol (t-BuOH) solution containing lipids dissolved therein (YSK13-C3/cholesterol/PEG-DMG = 50/50/3, 7.5 mM) was mixed with 200 µL of ultrapure water containing 80 µg siRNA dissolved therein, to which 2 mL of 20 mM citrate buffer (pH 4.0) was then all injected under vigorous stirring. The resulting mixture was diluted with D-PBS(-) and then centrifuged at 25°C at 1,000 g for 23 minutes using a ultrafiltration filter unit (Amicon Ultra-15, MWCO 50 kDa). After addition of 10 mL of D-PBS(-), the mixture was centrifuged again under the same conditions to obtain siRNA-loaded lipid nanoparticles (LNPs).

ICR mice (4 weeks of age, male) were intravenously administered with siRNA-loaded LNPs at a dose of 1 to 7 mg siRNA/kg, and blood was collected after 24 hours to obtain plasma. Plasma ALT/AST levels were measured with an ALT/AST assay kit (transaminase CII-Test Wako) according to the protocol attached thereto.

To the 90% tert-butanol (t-BuOH) solution containing lipids dissolved therein, DiD was added to account for 0.5 mol% of the total lipid content, followed by preparation of LNPs as described above to obtain DiD-labeled LNPs.

ICR mice (4 weeks of age, male) were intravenously administered with siRNA-loaded LNPs at a dose of 1 to 7 mg siRNA/kg, and livers, spleens, lungs and kidneys were collected after 30 minutes. Each organ was chopped and homogenized using a Micro Smash MS-100R in 10 volumes (based on tissue wet weight) of a 1% SDS solution. The fluorescence intensity of DiD in the homogenate solution was measured with an Enspire 2300 Multilabel Reader (PerkinElmer) (excitation wavelength: 650 nm, fluorescence wavelength: 680 nm). The amount of transfer to each tissue per dose was calculated from a calibration curve.

ICR mice (4 weeks of age, male) were intravenously administered with siRNA-loaded LNPs at a dose of 7 mg siRNA/kg, and livers were collected after 30 minutes. FITC-conjugated isolectin B4 was diluted to 50 µg/mL with PBS(-), to which the collected livers were then introduced and stained for 20 minutes under ice cooling. After washing with cold PBS(-), the livers were observed under a Nikon A1 microscope. Blood vessels are shown in green, and LNPs are shown in blue.

As a result, about 90% of LNPs were found to accumulate in the liver rapidly after administration (the lower-left panel of Figure 1).

However, high levels of ALT and AST indicative of hepatotoxicity were observed in high dose administration (the upper-right panel of Figure 1).

Moreover, LNPs were found to be also taken up by blood vessel cells in the liver, which served as a factor for hepatotoxicity (the lower-right panel of Figure 1).

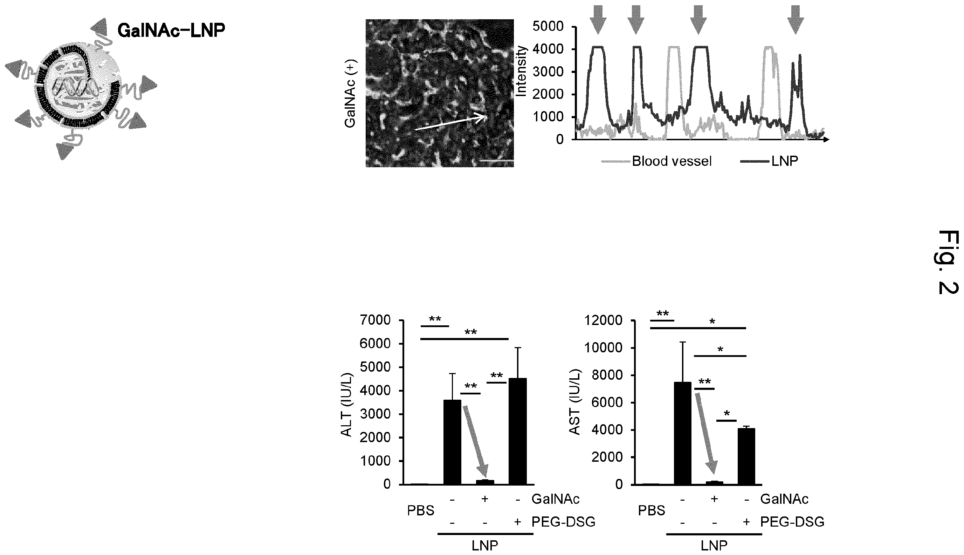

Figure 2 shows improvement to reduce hepatotoxicity in hepatic parenchymal cell-directed pH-responsive lipid nanoparticles.

400 µL of a 90% tert-butanol (t-BuOH) solution containing lipids dissolved therein (YSK13-C3/cholesterol/PEG-DMG/GalNAc-PEG-DSG = 50/50/2.5/0.5, 7.5 mM) was mixed with 200 µL of ultrapure water containing 80 µg of siRNA dissolved therein, to which 2 mL of 20 mM citrate buffer (pH 4.0) was then all injected under vigorous stirring (wherein "GalNAc" refers to N-acetyl-D-galactosamine). The resulting mixture was diluted with D-PBS(-) and then centrifuged at 25°C at 1,000 g for 23 minutes using a ultrafiltration filter unit (Amicon Ultra-15, MWCO 50 kDa). After addition of 10 mL of D-PBS(-), the mixture was centrifuged again under the same conditions to obtain siRNA-loaded GalNAc-modified LNPs. To the 90% tert-butanol (t-BuOH) solution containing lipids dissolved therein, DiD was added to account for 0.5 mol% of the total lipid content, thereby obtaining DiD-labeled GalNAc-modified LNPs.

ICR mice (4 weeks of age, male) were intravenously administered with GalNAc-modified LNPs at a dose of 7 mg siRNA/kg, and blood was collected after 24 hours to obtain plasma. Plasma ALT/AST levels were measured with an ALT/AST assay kit (transaminase CII-Test Wako) according to the protocol attached thereto.

ICR mice (4 weeks of age, male) were intravenously administered with DiD-labeled GalNAc-modified LNPs at a dose of 7 mg siRNA/kg, and livers were collected after 30 minutes. FITC-conjugated isolectin B4 was diluted to 50 µg/mL with PBS(-), to which the collected livers were introduced and stained for 20 minutes under ice cooling. After washing with cold PBS(-), the livers were observed under a Nikon A1 microscope.

As a result, GalNAc-modified LNPs showed reduced non-specific uptake to blood vessels (vascular endothelial cells), whereby specificity for hepatic parenchymal cells was improved (the left panel of Figure 2).

In addition, GalNAc modification caused no increases in plasma ALT and AST levels (the right panel of Figure 2).

Figure 3 shows the results indicating that GalNAc-LNP/pIC inhibited HBV replication in chimeric mice.

Figure 4 shows the results indicating that GalNAc-LNP/pIC reduces liver cccDNA levels in chimeric mice.

Figure 5 shows the results indicating that GalNAc-LNP/pIC induces IFNλ in chimeric mouse livers.

Figure 6 shows the results indicating that GalNAc-LNP/pIC induces IFNλ expression in hepatic parenchymal cells.

Figure 7 shows the results indicating that INEλ reduces cccDNA levels.

Figure 8 shows the results indicating that GalNAc-LNP/pIC administration enhances the expression of IRF 1 and IPNλ in chimeric mouse livers.

Figure 9 shows the results indicating that IRF 1 leads to a reduction in cccDNA levels.

Figure 10 shows the results indicating that the expression of Dock11 is reduced in GalNAc-LNP/pIC-administered chimeric mouse livers.

Figure 11 shows that IRF1 is involved in the regulation of Dock11 expression.

Figure 12A shows a schematic view of the construction of each vector for promoter analysis.

Figure 12B shows the results indicating that IRF1 regulates the promoter activity of Dock11.

Figure 13 shows the effect of the combined use of GalNAc-LNP/pIC and entecavir.

### Description of Embodiments

The present invention will be described in more detail below. The scope of the present invention is not limited by the following descriptions, and any embodiments other than those illustrated below may also be carried out with appropriate modifications without departing from the spirit of the invention. It should be noted that this specification incorporates the specification of Japanese Patent Application No. 2021-183329 (filed on November 10, 2021) in its entirety, based on which the present application claims priority. Moreover, all publications cited herein, including prior art documents, patent gazettes and other patent documents, are incorporated herein by reference.

### 1. Summary of the present invention

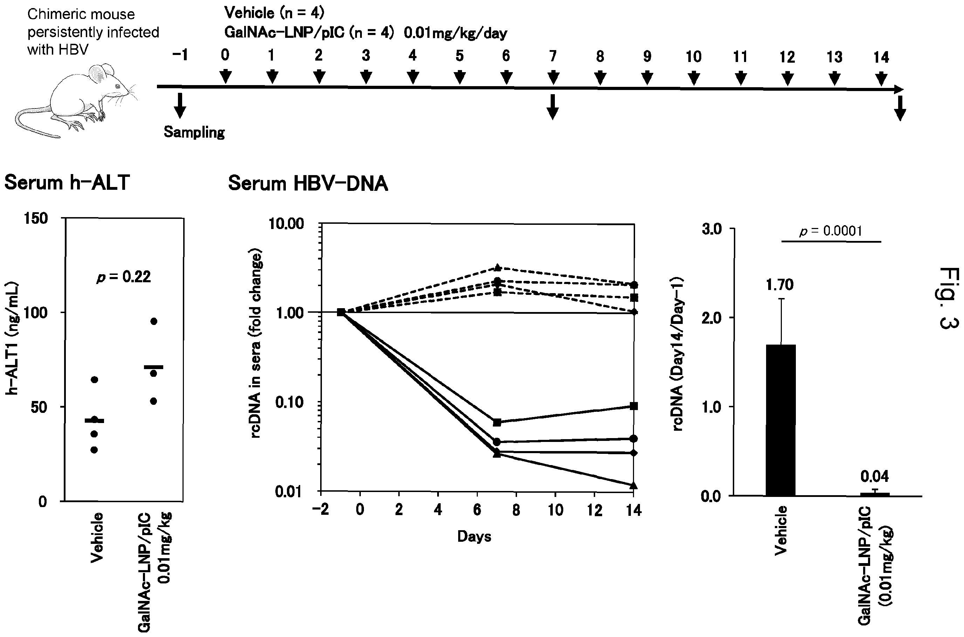

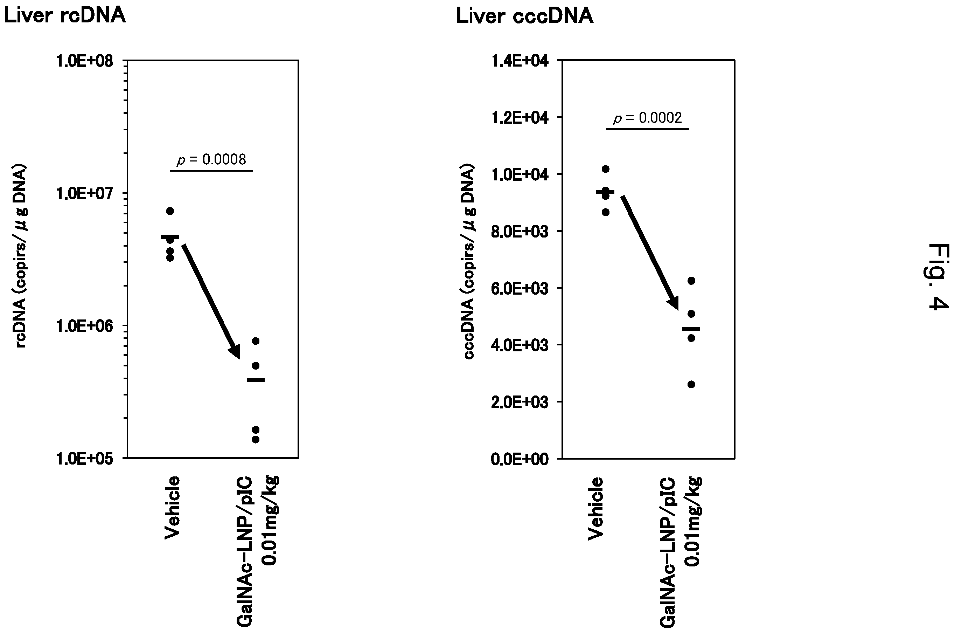

Among refractory viral infections, hepatitis B virus (HBV) known as a representative cause thereof is stably maintained in the form of covalently closed circular DNA (cccDNA) structure within the nuclei of hepatic parenchymal cells, thereby resulting in persistent infection. Thus, the disappearance of cccDNA is required for virus elimination from the infected cells. However, reverse transcriptase inhibitors (nucleos(t)ide analogues) conventionally used for treatment are incapable of effectively reducing cccDNA. For this reason, withdrawal from the therapeutic agents has become an important problem. The object of the present invention is to reduce or eliminate cccDNA levels by eliciting natural immune responses in a manner specific to hepatic parenchymal cells. To achieve this object, the inventors of the present invention have first prepared a complex encapsulating poly-IC (polyinosinic-polycytidylic acid) within a lipid nanoparticle (LNP). When this complex was administered to human liver chimeric mice persistently infected with HBV, a reduction in the viral load was observed. However, if the dose is slightly too high, strong hepatotoxicity will be elicited, so that the above complex cannot be provided for practical use. Upon analysis on the cause of this hepatotoxicity, the inventors of the present invention have elucidated that the above complex invades vascular endothelial cells to cause cell damage, which in turn leads to hepatotoxicity (Figure 1). Then, the inventors of the present invention have prepared a complex comprising N-acetyl-D-galactosamine (GalNAc) introduced onto the surface of LNP (Figure 2), with the aim that poly-IC encapsulated within this complex (within LNP) is specifically introduced into hepatocytes via the asialoglycoprotein receptor (ASGPR) present on the hepatocyte surface (Figure 3, Figure 4). As a result, the inventors of the present invention have succeeded in significantly reducing serum HBV load and liver HBV and cccDNA persistently infected in human liver chimeric mice without eliciting hepatotoxicity (Figure 3, Figure 4). Based on the above results, the inventors of the present invention have found that such a complex may be used as a therapeutic agent for refractory viral infections. This finding led to the completion of the present invention.

It should be noted that in the context of the present invention, "I" is intended to mean inosinic acid, while "C" is intended to mean cytidylic acid. Likewise, poly-I analogs and poly-C analogs are well known to those skilled in the art, but they are intended to mean those modified at all or part of the sugar, nucleobase or phosphate backbone portions which constitute a nucleic acid, with the aim of enhanced efficacy and improved stability, etc.

### 2. Therapeutic agent for refractory viral infections

The therapeutic agent for refractory viral infections according to the present invention (also referred to as the "pharmaceutical composition for treating refractory viral infections"; hereinafter collectively referred to as "the therapeutic agent of the present invention") is characterized by comprising a complex encapsulating poly-I or a poly-I analog and poly-C or a poly-C analog within a drug carrier useful for drug delivery into cells (hereinafter simply referred to as the "drug carrier"), wherein the surface of the drug carrier is conjugated with a molecule imparting the ability to accumulate in hepatocytes (hereinafter also referred to as "the complex of the present invention"), as described above.

In the therapeutic agent of the present invention, refractory viral infections to be treated are not limited to inflammations, symptoms and diseases which occur directly upon virus infection, and also include various symptoms and diseases, etc., which are then caused by these inflammations. Preferred examples of refractory viral infections include, but are not limited to, hepatitis virus infections, etc., and specific examples include various inflammations, symptoms and diseases falling within hepatitis, as well as cirrhosis and various types of liver cancer. The hepatitis virus intended here includes hepatitis B virus (HBV), etc. In more detail, the genotype of HBV is typically exemplified by type C.

The "drug carrier" used in the present invention is not limited in any way as long as it is pharmaceutically acceptable, is capable of encapsulating synthetic RNA, and has the ability to deliver the encapsulated synthetic RNA into cells. Such a drug carrier may be exemplified by lipid nanoparticles, cationic liposomes, atelocollagen, and polymeric micelles.

Specific examples of lipid nanoparticles may include those comprising cationic lipid YSK13-C3 (2-(dimethylamono)propyl (12Z,15Z)-3-((9Z,12Z)-octadeca-9,12-dien-1-yl)henicosa-2,12,15-trienoate) described in J. Control Release 266: 216-225, 2017, and those comprising cationic lipids described in J. Control Release 295: 140-152, 2019, particularly cationic CL4H6 (7-(4-(dipropylamino)butyl)-7-hydroxytridecane-1,13-diyl dioleate).

Likewise, examples of cationic liposomes may include oligofectamine^{®}, lipofectin^{®}, lipofectamine^{®}, lipofectamine 2000^{®}, lipofectace^{®}, DMRIE-C^{®}, GeneSilencer^{®}, TransMessenger^{®}, TransIT TKO^{®}, and the drug carrier disclosed in WO94/19314, i.e., a drug carrier formed essentially from a compound represented by the following general formula [I] [e.g., 2-O-(2-diethylaminoethyl)carbamoyl-1,3-O-dioleoyl glycerol, 3-O-(4-dimethylaminobutanoyl)-1,2-O-dioleyl glycerol, 3-O-(2-dimethylaminoethyl)carbamoyl-1,2-O-dioleyl glycerol, or 3-O-(2-diethylaminoethyl)-carbamoyl-1,2-O-dioleyl glycerol] and a phospholipid (hereinafter referred to as "the glycerol carrier of the present invention"). 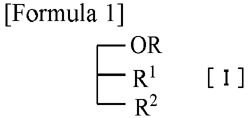 [in formula [I], R¹ and R² are different and each represent OY or -A-(CH₂)n-E, and n represents an integer of 0 to 4, wherein E represents pyrrolidino, piperidino, substituted or unsubstituted piperazino, morpholino, substituted or unsubstituted guanidino, or a group represented by the following formula: 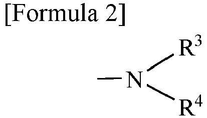 (wherein R³ and R⁴ are the same or different and each represent a hydrogen, a lower alkyl containing 1 to 4 carbon atoms, a hydroxy-lower alkyl containing 1 to 4 carbon atoms, or a mono- or di-lower alkylaminoalkyl (containing 2 to 8 carbon atoms)), and A represents any of groups represented by the following formulae: 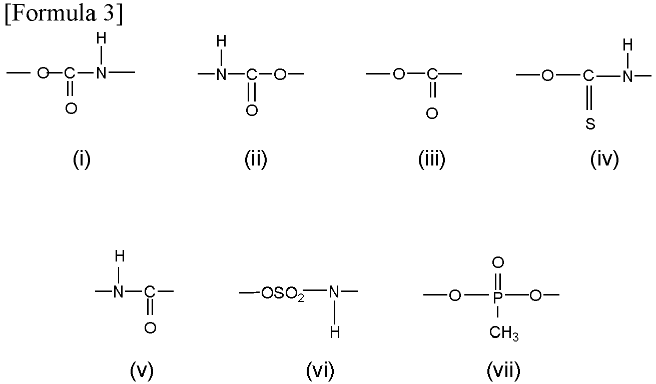 (i.e., any of the groups shown in (i) to (vii)). Moreover, R and Y are the same or different and each represent a saturated or unsaturated aliphatic hydrocarbon group containing 10 to 30 carbon atoms, or a saturated or unsaturated fatty acid residue containing 10 to 30 carbon atoms.]

The phospholipid in the glycerol carrier of the present invention is not limited in any way as long as it is a pharmaceutically acceptable phospholipid, but specific examples may include phosphatidylcholine, phosphatidylethanolamine, phosphatidylinositol, phosphatidylserine, sphingomyelin, lecithin and so no. Other examples may include hydrogenated phospholipids. Preferred phospholipids may include egg yolk phosphatidylcholine, egg yolk lecithin, soybean lecithin, and egg yolk phosphatide. It is possible to use one or more of these phospholipids.

Further, examples of polymeric micelles may include micelles composed of one or more materials, in any combination, selected from lactic acid-glycolic acid copolymer (PLGA), polylactic acid (PLA), chitosan, poly-L-lysine (PLL), polyethyleneimine (PEI), poly(2-dimethylamino)ethyl methacrylate (pDMAEMA), polyamidoamine (PAMAM), poly(β-amino ester) (PBAE), and polyethylene glycol adducts thereof.

Poly-I analogs and poly-C analogs are not limited in any way as long as they do not significantly impair the function of the original nucleic acid (e.g., poly-I in the case of poly-I analogs), but specific examples may include poly(7-deazainosinic acid), poly(2'-azidoinosinic acid), poly(cytidine-5'-thiophosphoric acid), poly(1-vinylcytidylic acid), poly(cytidylic acid, uridylic acid) copolymer, and poly(cytidylic acid, 4-thiouridylic acid) copolymer.

The chain lengths of poly-I, poly-I analogs, poly-C and poly-C analogs are not limited in any way, but they are each independently in the range of 50 to 2,000 bases, preferably in the range of 100 to 600 bases, and more preferably in the range of 200 to 500 bases. The effect of the present invention may be obtained if the chain length is less than 50 bases or greater than 2,000 bases. However, in some cases, a chain length of less than 50 bases may cause a problem in efficacy, while a chain length of greater than 2,000 bases may cause toxicity.

It should be noted that synthetic RNAs such as poly-I and poly-C are generally present with a certain distribution consisting of various chain lengths, so that the above chain length means the maximal distribution number of bases.

The component ratio between the drug carrier and synthetic RNAs such as poly-I and poly-C in the complex of the present invention will vary depending on the type of drug carrier used, the type and chain length of synthetic RNAs, and/or the type of virus and the degree of its multiplication, etc. However, relative to 10 parts by weight of the drug carrier, 0.05 to 10 parts by weight of synthetic RNAs is appropriate, preferably 0.1 to 4 parts by weight, and more preferably 0.3 to 2 parts by weight.

The complex of the present invention is designed such that the surface of the drug carrier is conjugated with a molecule imparting the ability to accumulate in hepatocytes. The molecule imparting the ability to accumulate in hepatocytes is not limited in any way, but preferred examples include N-acetylgalactosamine, galactose, and lactose, etc. N-Acetylgalactosamine is preferably in the D-form (i.e., N-acetyl-D-galactosamine). When conjugated to the surface of the drug carrier in this way, the molecule imparting the ability to accumulate in hepatocytes can prevent the complex of the present invention from invading vascular endothelial cells and causing cell damage, whereby natural immune responses can be elicited in a manner specific to hepatic parenchymal cells.

The component ratio between the drug carrier and the molecule imparting the ability to accumulate in hepatocytes in the complex of the present invention will vary depending on the type of drug carrier used and/or the type of molecule imparting the above accumulation ability, etc. However, relative to 100 parts by weight of the drug carrier, 0.1 to 30 parts by weight of the molecule imparting the above accumulation ability is appropriate, preferably 0.5 to 20 parts by weight, and more preferably 1 to 10 parts by weight.

In a preferred embodiment of the therapeutic agent of the present invention, when the drug carrier is a lipid nanoparticle, the therapeutic agent may comprise a complex encapsulating poly-I or a poly-I analog and poly-C or a poly-C analog, each synthetic RNA having a chain length in the range of 50 to 1000 bases (particularly preferably 100 to 400 bases), within a lipid nanoparticle formed essentially from a cationic lipid, cholesterol, a PEG lipid and so on, wherein the surface of the drug carrier is conjugated with N-acetyl-D-galactosamine.

Alternatively, in another preferred embodiment of the therapeutic agent of the present invention, when the drug carrier is a cationic liposome, the therapeutic agent may comprise a complex encapsulating poly-I or a poly-I analog and poly-C or a poly-C analog, each synthetic RNA having a chain length in the range of 50 to 1000 bases (particularly preferably 100 to 400 bases), within a cationic liposome formed essentially from 2-O-(2-diethylaminoethyl)carbamoyl-1,3-O-dioleoyl glycerol and lecithin as a phospholipid, wherein the surface of the drug carrier is conjugated with N-acetyl-D-galactosamine.

### 3. INFλ inducer, IRF1 expression enhancer, and Dock11 expression inhibitor

The INFλ inducer according to the present invention (hereinafter referred to as "the inducer of the present invention") is characterized by comprising a complex encapsulating poly-I or a poly-I analog and poly-C or a poly-C analog within a drug carrier useful for drug delivery into cells, wherein the surface of the drug carrier is conjugated with a molecule imparting the ability to accumulate in hepatocytes, as described above. The inducer of the present invention preferably induces IFNλ in hepatocytes.

The IRF1 expression enhancer according to the present invention (hereinafter referred to as "the enhancer of the present invention") is characterized by comprising a complex encapsulating poly-I or a poly-I analog and poly-C or a poly-C analog within a drug carrier useful for drug delivery into cells, wherein the surface of the drug carrier is conjugated with a molecule imparting the ability to accumulate in hepatocytes, as described above. The enhancer of the present invention preferably enhances IRF1 expression in hepatocytes.

The Dock11 expression inhibitor according to the present invention (hereinafter referred to as "the inhibitor of the present invention") is characterized by comprising a complex encapsulating poly-I or a poly-I analog and poly-C or a poly-C analog within a drug carrier useful for drug delivery into cells, wherein the surface of the drug carrier is conjugated with a molecule imparting the ability to accumulate in hepatocytes, as described above. The inhibitor of the present invention preferably inhibits Dock11 expression in hepatocytes.

As to details such as the components constituting the inducer of the present invention, the enhancer of the present invention and the inhibitor of the present invention, the explanations about the therapeutic agent of the present invention given in 2. above can all be applied.

### 4. Form of use of the therapeutic agent of the present invention and the inducer and others of the present invention

The therapeutic agent of the present invention, as well as the inducer of the present invention, the enhancer of the present invention and the inhibitor of the present invention (hereinafter referred to as "the inducer and others of the present invention") may be in the form of, for example, solutions (e.g., injections, drops) or lyophilized formulations thereof.

The therapeutic agent of the present invention and the inducer and others of the present invention may comprise appropriate amounts of pharmaceutically acceptable additives and excipients of any type, for example, an emulsifier aid, a stabilizing agent, an isotonizing agent, and/or a pH adjuster. Specific examples may include emulsifier aids such as fatty acids containing 6 to 22 carbon atoms (e.g., caprylic acid, capric acid, lauric acid, myristic acid, palmitic acid, stearic acid, oleic acid, linoleic acid, arachidonic acid, docosahexaenoic acid) and pharmaceutically acceptable salts thereof (e.g., sodium salt, potassium salt, calcium salt), albumin, and dextran; stabilizing agents such as cholesterol and phosphatidic acid; isotonizing agents such as sodium chloride, glucose, maltose, lactose, sucrose, and trehalose; and pH adjusters such as hydrochloric acid, sulfuric acid, phosphoric acid, acetic acid, sodium hydroxide, potassium hydroxide, and triethanolamine, etc.

Moreover, the therapeutic agent of the present invention and the inducer and others of the present invention may further comprise a pharmaceutically acceptable additional therapeutic agent for viral infections, etc., or may be used in combination with such an additional therapeutic agent for viral infections, etc., without being limited thereto. Such an additional therapeutic agent for viral infections, etc. may be contained or used as appropriate in an amount not to significantly impair the effect of the therapeutic agent of the present invention and the inducer and others of the present invention. Examples of such an additional therapeutic agent for viral infections, etc. include, but are not limited to, nucleos(t)ide analogues (e.g., entecavir, tenofovir, lamivudine, and adefovir), PEG-IFNα, and PEG-IFNλ, as well as prodrugs thereof, which may be used either alone or in combination. For example, prodrugs of tenofovir may be exemplified by tenofovir alafenamide fumarate, and tenofovir disoproxil fumarate, etc.

The therapeutic agent of the present invention and the inducer and others of the present invention may be prepared, e.g., by mixing, stirring or dispersing the drug carrier or its source compound(s) and synthetic RNAs such as poly-I and poly-C in a standard manner. For example, they may be prepared in the same manner as commonly used for preparation of lipid nanoparticles or liposomes. In more detail, the drug carrier or its source compound(s) and, for example, double-stranded poly-I poly-C (i.e., poly-IC (polyinosinic-polycytidylic acid)) or single-stranded poly-I and single-stranded poly-C may be dispersed in an aqueous solution with an appropriate dispersing device. Alternatively, the source compound(s) may first be dispersed to form a drug carrier, followed by addition of, for example, double-stranded poly-I poly-C or single-stranded poly-I and single-stranded poly-C, and the resulting mixture may be dispersed again. In this case, for example, a molecule imparting the ability to accumulate in hepatocytes (e.g., N-acetylgalactosamine) may be conjugated in advance with a molecule serving as a source compound of the drug carrier, and the resulting conjugate may be used for drug carrier formation to thereby achieve a state where the surface of the formed drug carrier is conjugated with the molecule imparting the ability to accumulate in hepatocytes.

Examples of an aqueous solution used in the dispersing process may include water for injection, distilled water for injection, an electrolyte solution (e.g., physiological saline), a glucose solution and so on. Examples of an appropriate dispersing device may include a homomixer, a homogenizer, an ultrasonic disperser, an ultrasonic homogenizer, a high-pressure emulsifying disperser, a Microfluidizer^{™}, a Nanomizer^{™}, an Altimizer^{™}, a DeBee^{™} homogenizer, and a Manton-Gaulin high-pressure homogenizer. Moreover, the dispersing process may also be accomplished in several divided stages, e.g., through a crude dispersing stage.

For use as a drug carrier, a commercially available product may be used in accordance with its instructions, or may be processed appropriately before use.

Pharmaceutically (pharmacologically) acceptable additives of any type may be added at an appropriate step either before or after dispersing.

Lyophilized formulations of the therapeutic agent of the present invention and the inducer and others of the present invention may be prepared in a standard manner. For example, the therapeutic agent of the present invention and the inducer and others of the present invention in liquid form may be sterilized and dispensed in predetermined volumes into vial bottles. Then, under conditions of about -40°C to -20°C, the vial bottles may be subjected to preliminary freezing for about 2 hours, followed by primary dying at about 0°C to 10°C under reduced pressure and further secondary drying at about 15°C to 25°C under reduced pressure for lyophilization. Subsequently, the inside of each vial may usually be purged with a nitrogen gas, and then sealed to obtain lyophilized formulations of the therapeutic agent of the present invention and the inducer and others of the present invention.

The lyophilized formulations of the therapeutic agent of the present invention and the inducer and others of the present invention may usually be reconstituted before use by addition of any appropriate solution (reconstitution solution). Examples of such a reconstitution solution may include water for injection, an electrolyte solution (e.g., physiological saline), a glucose solution, and other commonly-used infusion solutions. The volume of this reconstitution solution will vary depending on the purpose of use, etc., and is not limited in any way, but an appropriate volume is 0.5 to 2 times the volume before lyophilization or is 500 mL or less.

The therapeutic agent of the present invention is expected to be used against various viruses which may cause refractory viral infections, for example, hepatitis B virus and others, without being limited thereto. The antiviral effect of the therapeutic agent of the present invention can last throughout the period of administration. As is evident from the Example section described later, the therapeutic agent of the present invention significantly reduces, for example, serum HBV load and liver HBV and cccDNA persistently infected in human liver chimeric mice. In addition, the therapeutic agent of the present invention induces IFNλ expression in hepatocytes, and also enhances IRF1 expression, and further inhibits Dock11 expression (or reduces Dock11 expression). These effects are all related to significantly reducing cccDNA levels in hepatocytes.

Thus, the therapeutic agent of the present invention may also encompass a therapeutic agent for refractory viral infections (e.g., hepatitis B) and a pharmaceutical composition for treating these infections, each comprising IFNλ and/or IRF1.

Further, the present invention also provides an agent for reducing DNA levels of refractory viruses (e.g., hepatitis B virus) or cccDNA levels thereof, and a method for reducing DNA levels of refractory viruses (e.g., hepatitis B virus) or cccDNA levels thereof, which comprises allowing IFNλ and/or IRF1 to act on hepatocytes or allowing IFNλ and/or IRF1 to be expressed in hepatocytes. It should be noted that any technique may be used for allowing IFNλ and/or IRF1 to act on hepatocytes, and known procedures for drug administration to the liver and thus to hepatocytes may be used for this purpose. Likewise, any technique may be used for allowing IFNλ and/or IRF1 to be expressed in hepatocytes, and known gene recombination technology or gene transfer and expression technology may be used for this purpose.

The therapeutic agent of the present invention and the inducer and others of the present invention are effective for animals including humans, and are particularly effective when allowed to act on hepatocytes.

The therapeutic agent of the present invention and the inducer and others of the present invention may be administered, for example, by intravenous administration, subcutaneous administration, hepatic intraarterial administration, topical administration (e.g., transmucosal administration, transnasal administration, inhalation administration), etc.

The dose of the therapeutic agent of the present invention and the inducer and others of the present invention will vary depending on the type of drug carrier used, the type, composition and chain length of synthetic RNAs, the type of virus and the stage of progress, the age of a patient, differences among animal species, the route of administration, the mode of administration, etc. However, the single dose of synthetic RNAs such as poly-I and poly-C is usually appropriately 1 µg to 50 mg/human, and preferably 10 µg to 10 mg/human. The therapeutic agent of the present invention and the inducer and others of the present invention may be administered once to three times a day, e.g., every day, every other day, every week or every 2 weeks by one-shot administration or drip infusion administration, etc.

The present invention may also provide a method for treating refractory viral infections, which comprises administering the therapeutic agent of the present invention described above to patients with refractory viral infections, the use of the therapeutic agent of the present invention for the treatment of refractory viral infections, and the use of the complex of the present invention for the manufacture of the therapeutic agent for refractory viral infections. The therapeutic agent of the present invention may also be used as a prophylactic agent (prophylactic pharmaceutical composition) for refractory viral infections.

The present invention may also provide a method for inducing INFλ, which comprises allowing the inducer of the present invention to act on hepatocytes, the use of the inducer of the present invention for the induction of INFλ in hepatocytes, and the use of the complex of the present invention for the manufacture of the INFλ inducer.

The present invention may also provide a method for enhancing IRF1 expression, which comprises allowing the enhancer of the present invention to act on hepatocytes, the use of the enhancer of the present invention for the enhancement of IRF1 expression in hepatocytes, and the use of the complex of the present invention for the manufacture of the IRF1 expression enhancer.

The present invention may also provide a method for inhibiting Dock11 expression, which comprises allowing the inhibitor of the present invention to act on hepatocytes, the use of the inhibitor of the present invention for the inhibition of Dock11 expression in hepatocytes, and the use of the complex of the present invention for the manufacture of the Dock11 expression inhibitor.

### Examples

The present invention will be further described in more detail by way of the following examples, although the present invention is not limited thereto.

### [Example 1]

### <Method>

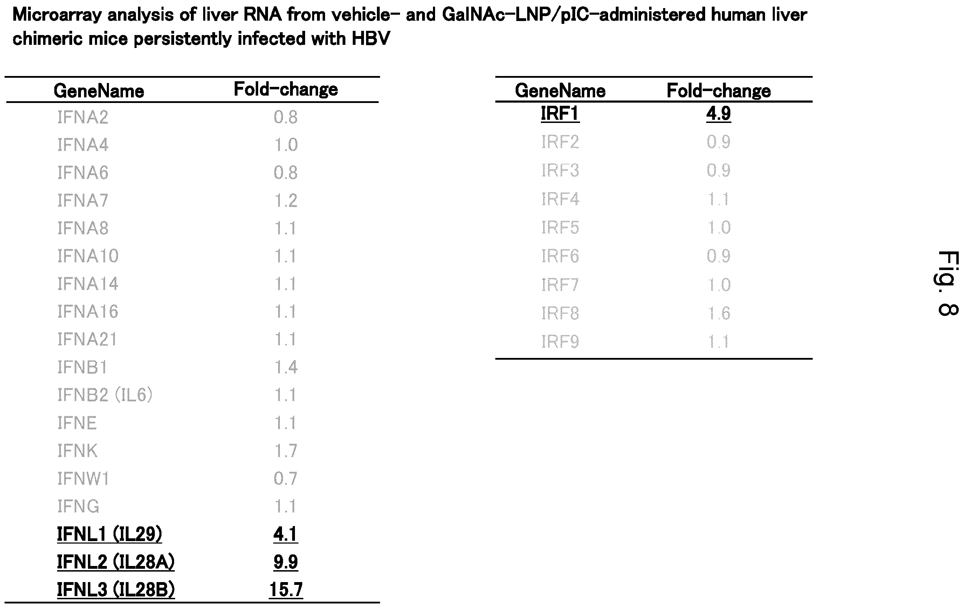

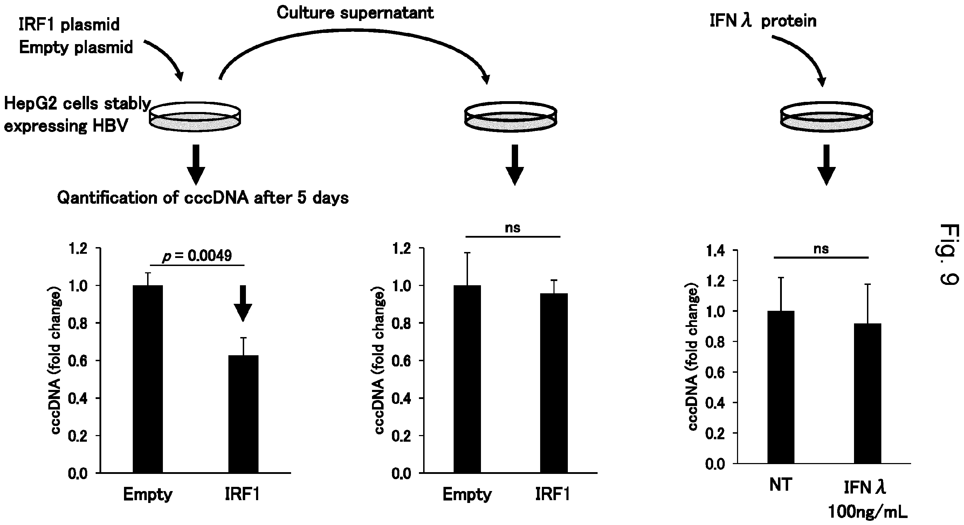

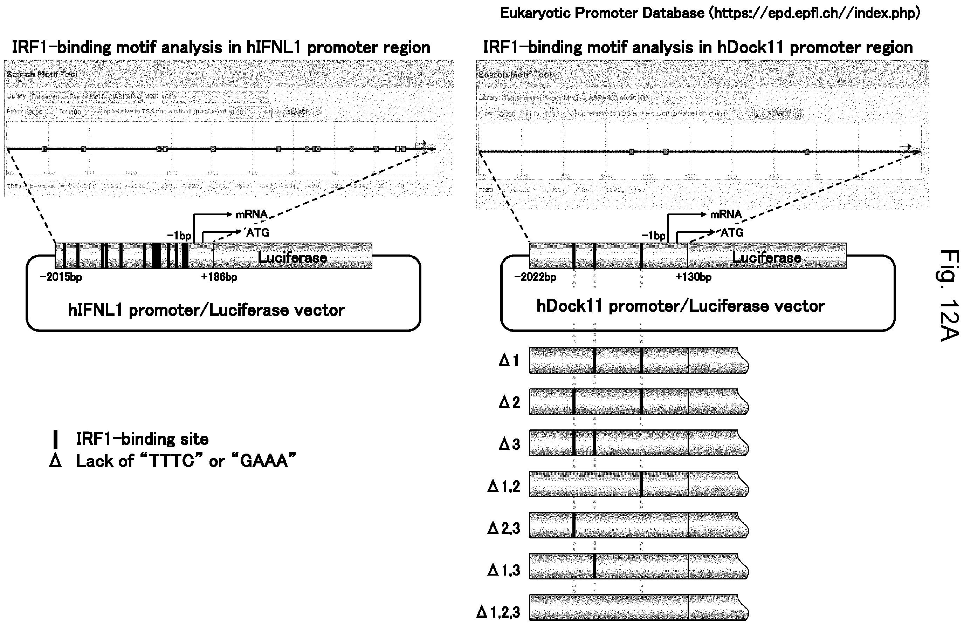

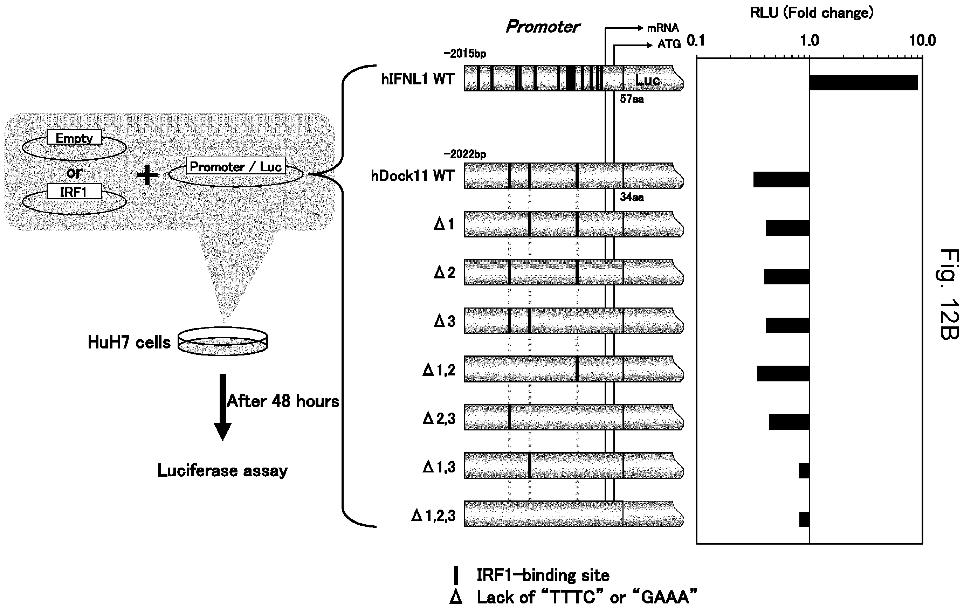

"GalNAc-LNP/pIC" was prepared as a complex encapsulating poly-IC within a hepatic parenchymal cell-directed lipid nanoparticle. In more detail, N-acetyl-D-galactosamine (GalNAc) was introduced onto the surface of the lipid nanoparticle to prepare "GalNAc-LNP/pIC" which allows poly-IC encapsulated within the lipid nanoparticle to be specifically introduced into hepatocytes via the asialoglycoprotein receptor (ASGPR) present on the surface of the hepatocytes. GalNAc-LNP/pIC was intravenously administered to human liver chimeric mice persistently infected with HBV, and evaluated for its hepatotoxicity and also examined for its effect on liver cccDNA (Figure 3). Further, RNA microarray analysis was performed on livers from the chimeric mice (Figure 8). Primary human hepatocytes persistently infected with HBV and HepG2 cells stably expressing HBV were used to analyze the mechanism of GalNAc-LNP/pIC-induced reduction in cccDNA levels (Figure 9, Figure 10, Figure 11). The hIFNL1 promoter (-2015 to +186 bp) and the hDock11 promoter (-2022 to +130 bp) were each amplified with Q5 High-Fidelity DNA Polymerase (New England Biolabs) by using primary human hepatocyte gDNA as a template, and then inserted upstream of the Firefly Luciferase gene in the pGL4.51 vector (Promega) in such a way as to form a fusion protein with Firefly Luciferase. The N-terminal 57 amino acids of IFNλ1 protein or the N-terminal 34 amino acids of Dock11 protein are linked in frame to the N-terminal end of Luciferase protein. A series of mutant vectors were constructed to lack three putative IRF1-binding sequences (-1285, -1121 and -453 bp) (TTTC or GAAA) in the Dock11 promoter (Figure 12A). HuH7 cells (1.6 × 10⁶ cells/well) were seeded in 48-well plates. On the following day, the promoter/Luciferase vectors (25 ng/well) were each transfected together with phRL-TK(Int-) (10 ng/well, Promega) using an IRF1 plasmid (250 ng/well) or an empty plasmid and Lipofectamine 3000 (Invitrogen). The cells after 48 hours were washed with PBS, and lysed with 70 µL of Cell Culture Lysis Reagent (Promega). According to the protocols of a Luciferase Assay System and a Renilla Luciferase Assay System (Promega), Firefly and Renilla Luciferase activities were measured with an EnVision reader (PerkinElmer). The value of Firefly Luciferase activity was normalized with the value of Renilla Luciferase activity. The Luciferase activity ratio of the IRF1 plasmid-transfected cells to the empty plasmid-transfected cells was shown in graphical form (Figure 12B).

### <Result>

GalNAc-LNP/pIC was found to significantly reduce serum HBV load and liver HBV and cccDNA persistently infected, without showing hepatotoxicity in the livers of human liver chimeric mice (Figure 3, Figure 4).

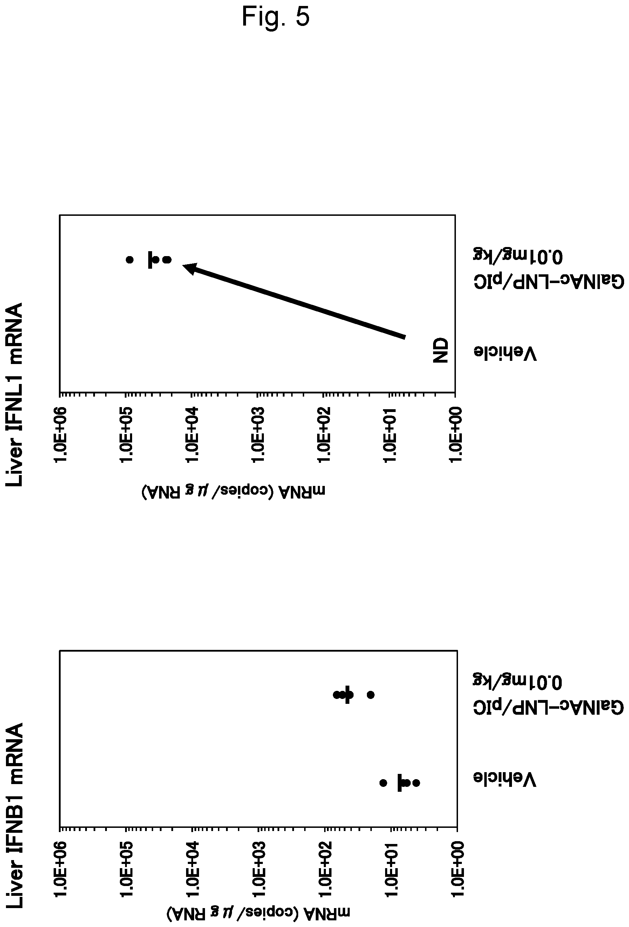

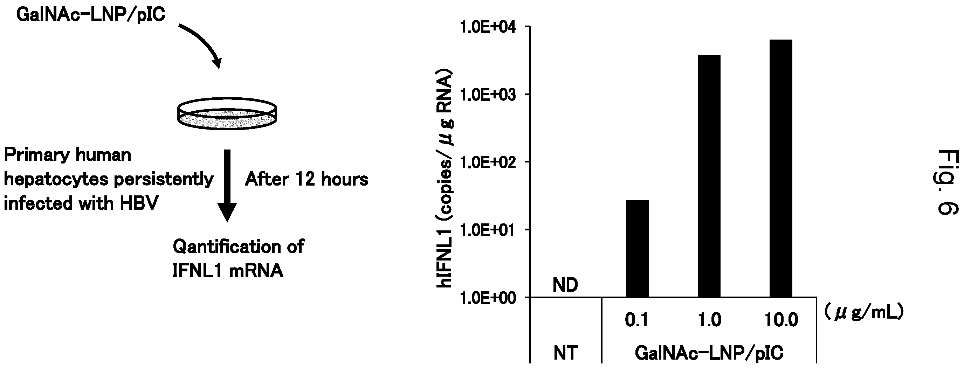

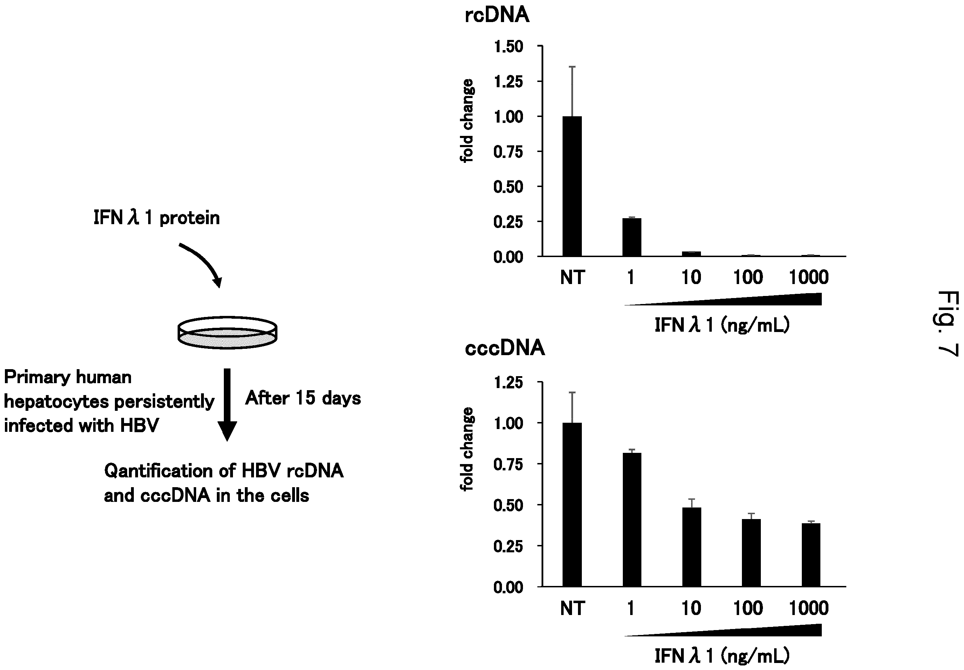

In this case, GalNAc-LNP/pIC induced IFNλ expression in hepatocytes to thereby significantly reduce cccDNA levels (Figure 5). However, there was little induction of IFNβ (Figure 5). This GalNAc-LNP/pIC-mediated IFNλ induction was also observed in *in vitro* primary human hepatocytes persistently infected with HBV (Figure 6). Then, IPNλ was added to primary human hepatocytes persistently infected with HBV, indicating that IPNλ significantly reduced cccDNA levels in these cells, as in the case of GalNAc-LNP/pIC administration (Figure 7).

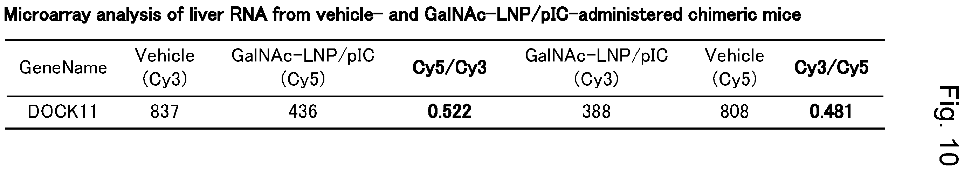

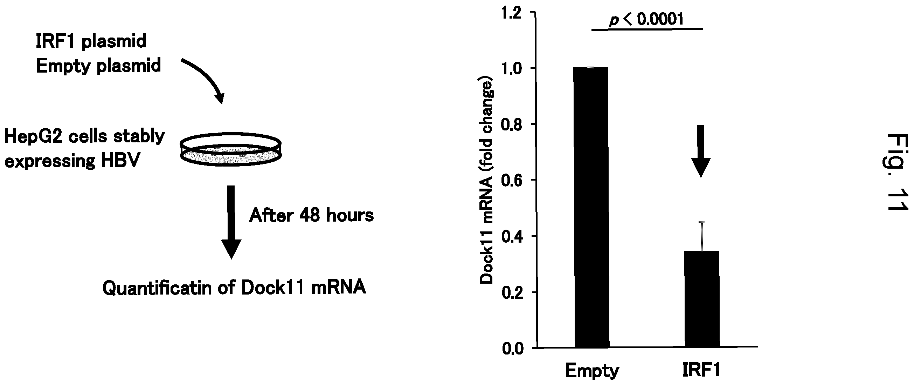

From the microarray analysis performed on livers from human liver chimeric mice receiving GalNAc-LNP/pIC administration, it was indicated that the expression of IRF1, a transcription factor regulating the expression of many genes involved in natural immune responses, and the expression of IFNλ were both enhanced (Figure 8). Then, an IRF1 expression plasmid was transfected into HepG2 cells stably expressing HBV, indicating that IRF1 significantly reduced cccDNA levels, as in the case of GalNAc-LNP/pIC administration (Figure 9). Further, from the microarray analysis performed on livers from human liver chimeric mice receiving GalNAc-LNP/pIC administration, it was found that the expression of Dock11 involved in the stable maintenance of cccDNA was reduced (Figure 10). IRF1 was involved in the reduced expression of Dock11 (Figure 11), thus indicating that HBV cccDNA levels were inhibitorily regulated by a pathway different from that of IFNλ. Namely, there were identified two cccDNA inhibitory pathways induced by GalNAc-LNP/pIC. Moreover, IRF1 was found to activate the IFNL1 promoter. On the other hand, IRF1 was found to inhibit the activity of the Dock11 promoter. From the experiments using the plasmids lacking IRF1-binding sequences, it was suggested that IRF1 would bind to and thereby negatively regulate the Dock11 promoter (Figure 12B).

### <Detailed explanation of each figure>

The figures shown in the above Method and Result sections will each be explained below in more detail.

### (1) Figure 3

### Method:

400 µL of a 90% tert-butanol (t-BuOH) solution containing lipids dissolved therein (YSK13-C3/cholesterol/PEG-DMG = 50/50/3, 7.5 mM) was mixed with 200 µL of physiological saline containing 80 µg of pIC (SIGMA, P1530) dissolved therein, to which 2 mL of 20 mM citrate buffer (pH 4.0) was then all injected under vigorous stirring. The resulting mixture was diluted with D-PBS(-) and then centrifuged at 25°C at 1,000 g for 23 minutes using a ultrafiltration filter unit (Amicon Ultra-15, MWCO 50 kDa). After addition of 10 mL of physiological saline, the mixture was centrifuged again under the same conditions and then adjusted to 750 µL by addition of physiological saline, thereby obtaining pIC-loaded LNPs (4 mM lipid). To 750 µL of a solution (20 mM MES buffer, 130 mM NaCl, 20% EtOH, pH 6.0) containing PEG-DSG and GalNAc-PEG-DSG at concentrations of 2.5 mol% and 0.5 mol%, respectively, relative to the total lipid content, the pIC-loaded LNPs were all added to conduct PEG modification reaction at 45°C at 800 rpm for 45 minutes. The reaction mixture was diluted with D-PBS(-) and then subjected again to ultrafiltration under the same conditions as shown above, thereby obtaining pIC-loaded GalNac-modified LNPs. Using a Zetasizer Nano ZS analyzer (Malvern Panalytical), the LNPs were measured for their particle size, polydispersity index (PDI) and zeta potential. The pIC-loaded GalNac-modified LNPs were found to have a particle size of 117 nm (zeta average), a PDI of 0.043 and a zeta potential of -0.97 mV. The recovery rate and encapsulation rate of pIC were 65.6% and 98.1%, respectively, as measured by Ribogreen assay.

The GalNAc-LNP/pIC administration test on human liver chimeric mice persistently infected with HBV was conducted by PhoenixBio Co., Ltd., Japan.

GalNAc-LNP/pIC encapsulating poly-IC within GalNAc-LNP was intravenously administered every day at a dose of 0.01 mg/kg/day for 14 days to human liver chimeric mice persistently infected with HBV

As a vehicle, PBS (GIBCO) was administered.

At 1, 7 and 14 days after administration, blood was collected to measure serum h-ALT and rcDNA (relaxed circular DNA).

The concentration of serum h-ALT1 was measured by ELISA assay in Institute of Immunology, Japan.

Serum DNA was extracted by the sodium iodide method. Serum (1 µL) was mixed with 200 µL of lysis buffer (10 mM EDTA, 500 mM 2-mercaptoethanol, 50 mM Tris-HCl, 75 mM KCl, 5% sarcosyl, 0.1 µg/µL protease K, pH 8.0), followed by heating at 55°C for 20 minutes.

After addition of 300 µL of a NaI solution (40 mM Tris-HCl, 20 mM EDTA, 7.6 M sodium iodide, coprecipitant), the mixture was allowed to stand at room temperature for 15 minutes. After addition of 600 µL of mixed alcohols (40% isopropanol, 60% 1-butanol), the mixture was cooled on ice for 15 minutes. The mixture was centrifuged at 4°C at 15,000 × g for 20 minutes to precipitate DNA. The DNA was washed twice with 70% ethanol and dissolved in TE buffer.

For measurement of rcDNA, the following primers and probe targeting HBV:
200 nM Forward primer (5'-CACATCAGGATTCCTAGGACC-3' (SEQ ID NO: 1)),
200 nM Reverse primer (5'-AGGTTGGTGAGTGATTGGAG-3' (SEQ ID NO: 2)),
300 nM Probe (5'FAM-CAGAGTCTAGACTCGTGGTGGACTTC-3'TAMRA (SEQ ID NO: 3))
were used, and an aliquot equivalent to 250 ng gDNA was measured according to the protocol of THUNDERBIRD Probe qPCR Mix (TOYOBO). Real-time PCR of rcDNA was performed using a CFX95 Real-time System (Bio-Rad) for 54 cycles of 50°C for 2 minutes, 95°C for 1 minute, and then 95°C for 20 seconds and 60°C for 1 minute.

### Result:

The mice receiving GalNAc-LNP/pIC administration showed no significant difference in their serum ALT compared to the mice receiving vehicle administration.

At 7 days after GalNAc-LNP/pIC administration, serum rcDNA was significantly reduced by 90% or more.

### (2) Figure 4

### Method:

rcDNA and cccDNA were quantified in livers from the human liver chimeric mice at 14 days after GalNAc-LNP/pIC or vehicle administration.

The frozen livers were each homogenized by adding 1 mL of homogenization buffer (0.14 M NaCl, 10 mM Tris-HCl pH 7.5). After addition of 1 mL of lysis buffer (25 mM EDTA, 0.3 M NaCl, 2% SDS, 0.2 M Tris-HCl pH 7.5) and 30 µL of protease K (20 mg/mL), the homogenate was digested overnight at 37°C. gDNA was isolated by phenol-chloroform treatment and collected by ethanol precipitation.

The gDNA was washed twice with 70% ethanol and dissolved at 50 ng/µL in TE buffer.

5 µL of the 50 ng/µL gDNA solution was treated for 1 hour with 10 U of Plasmid-Safe ATP-dependent DNase (Epicentre) according to the protocol, and used for cccDNA quantification.

For measurement of rcDNA, the following primers and probe targeting HBV:
200 nM Forward primer (5'-CACATCAGGATTCCTAGGACC-3' (SEQ ID NO: 1)),
200 nM Reverse primer (5'-AGGTTGGTGAGTGATTGGAG-3' (SEQ ID NO: 2)),
300 nM Probe (5'FAM-CAGAGTCTAGACTCGTGGTGGACTTC-3'TAMRA (SEQ ID NO: 3))
were used, and an aliquot equivalent to 250 ng gDNA was measured according to the protocol of THUNDERBIRD Probe qPCR Mix (TOYOBO).

For measurement of cccDNA,
300 nM Forward primer (5'-CAAGGCACAGCTTGGAGGCTTGAAC-3' (SEQ ID NO: 4)),
500 nM Reverse primer (5'-acggggcgcacctctctttacgcgg-3' (SEQ ID NO: 5)),
300 nM Probe (5'FAM-CCGTGTGCACTTCGCTTCACCTCTGC-3'TAMRA (SEQ ID NO: 6))
were used, and the Plasmid-Safe ATP-dependent DNase-treated product (equivalent to 50 ng total DNA) was measured according to the protocol of THUNDERBIRD Probe qPCR Mix (TOYOBO).

Real-time PCR of rcDNA was performed using a CFX95 Real-time System (Bio-Rad) for 54 cycles of 50°C for 2 minutes, 95°C for 1 minute, and then 95°C for 20 seconds and 60°C for 1 minute. Real-time PCR of cccDNA was performed for 54 cycles of 50°C for 2 minutes, 95°C for 5 minutes, and then 95°C for 20 seconds and 65°C for 1 minute.

### Result:

Upon GalNAc-LNP/pIC administration, liver rcDNA and cccDNA were each significantly reduced.

### (3) Figure 5

### Method:

Liver IFNB1 and IFNL1 mRNAs were each quantified by real-time RT-PCR at 14 days after GalNAc-LNP/pIC or vehicle administration.

The frozen livers were each homogenized in 1 mL of a guanidium thiocyanate solution (6 M guanidium thiocyanate, 37.5 mM sodium citrate, 0.75% lauroylsarcosine sodium salt, 1.4% 2-mercaptoethanol).

After addition of 27 µL of 3 M sodium acetate, the homogenate was mixed with 400 µL of acidic saturated phenol. The homogenate was mixed with 80 µL of chloroform and cooled on ice for 15 minutes, followed by centrifugation (at 4°C at 20000 × g for 20 minutes) to separate an aqueous layer (220 µL) which was then transferred to a new tube.

The aqueous layer was mixed with 22 µL of 3 M sodium acetate and 660 µL of 99% ethanol, and then cooled at -20°C for 2 hours. RNA was collected by centrifugation (at 4°C at 20000 × g for 10 minutes).

The collected RNA was purified again according to the protocol of an RNeasy Mini kit (QIAGEN).

IFNB1 and IFNL1 mRNAs were measured using TaqMan Gene Expression Assays (Thermo, Hs00277188_s1 and Hs00601677_g1) according to the protocol of ABI TaqMan EZ RT-PCR CORE REAGENTS (Applied Biosystems).

As an endogenous control, human actin beta mRNA (Applied Biosystems, 4352935E) was used to normalize the measured values.

Real-time RT-PCR was performed using a CFX95 Real-time System (Bio-Rad) for 50 cycles of 50°C for 2 minutes, 60°C for 30 minutes, 95°C for 5 minutes, and then 95°C for 20 seconds and 62°C for 1 minute.

### Result:

GalNAc-LNP/pIC administration induced only about a 6-fold increase in the expression of IFNB1 mRNA, and its expression level remained low.

On the other hand, IFNL1 mRNA in the mice receiving vehicle administration was less than the detection sensitivity, whereas IFNL1 mRNA was dramatically increased to 3.5 × 10⁴ copies/µg RNA upon GalNAc-LNP/pIC administration.

### (4) Figure 6

### Method:

GalNAc-LNP/pIC was administered to primary human hepatocytes persistently infected with HBV, and IFNL1 mRNA after 12 hours was quantified.

GalNAc-LNP/pIC was administered to primary human hepatocytes persistently infected with HBV for 22 days or longer (PhoenixBio Co., Ltd., Japan), and the cells were collected after 12 hours. RNA was extracted according to the protocol of ReliaPrep RNA Miniprep Systems (Promega).

cDNA was synthesized according to the protocol of SuperScript III Reverse Transcriptase (Invitrogen). IFNL1 mRNA was quantified using TaqMan Gene Expression Assays (Thermo, Hs00601677_g1) according to the protocol of TaqMan Universal Master Mix II, with UNG (Thermo). As an endogenous control, human actin beta mRNA (Applied Biosystems, 4352935E) was used to normalize the measured values.

Real-time PCR was performed using a CFX95 Real-time System (Bio-Rad) for 50 cycles of 50°C for 2 minutes, 95°C for 10 minutes, and then 95°C for 15 seconds and 60°C for 1 minute.

### Result:

The expression of IFNL1 mRNA was induced in a manner dependent on the dose of GalNAc-LNP/pIC.

### (5) Figure 7

### Method:

Primary human hepatocytes persistently infected with HBV were treated with IFNλ1 protein for 15 days, and rcDNA and cccDNA were quantified.

Primary human hepatocytes persistently infected with HBV for 22 days or longer (PhoenixBio Co., Ltd., Japan) were cultured in a medium containing recombinant human IFN-lambda 1 (R&D systems) with medium replacement every 5 days, and the cells were collected after 15 days.

DNA in the cells were extracted by the sodium iodide method.

The cells were mixed with 200 µL of lysis buffer (10 mM EDTA, 500 mM 2-mercaptoethanol, 50 mM Tris-HCl, 75 mM KCl, 5% sarcosyl, 0.1 µg/µL protease K, pH 8.0), followed by heating at 55°C for 20 minutes. After addition of 300 µL of a NaI solution (40 mM Tris-HCl, 20 mM EDTA, 7.6 M sodium iodide, coprecipitant), the mixture was allowed to stand at room temperature for 15 minutes. After addition of 600 µL of mixed alcohols (40% isopropanol, 60% 1-butanol), the mixture was cooled on ice for 15 minutes. The mixture was centrifuged at 4°C at 15,000 × g for 20 minutes to precipitate DNA. The DNA was washed twice with 70% ethanol and dissolved in TE buffer.

For measurement of rcDNA, the following primers and probe targeting HBV:
200 nM Forward primer (5'-CACATCAGGATTCCTAGGACC-3' (SEQ ID NO: 1)),
200 nM Reverse primer (5'-AGGTTGGTGAGTGATTGGAG-3' (SEQ ID NO: 2)),
300 nM Probe (5'FAM-CAGAGTCTAGACTCGTGGTGGACTTC-3'TAMRA (SEQ ID NO: 3))
were used, and an aliquot equivalent to 250 ng gDNA was measured according to the protocol of THUNDERBIRD Probe qPCR Mix (TOYOBO).

For measurement of cccDNA,
300 nM Forward primer (5'-CAAGGCACAGCTTGGAGGCTTGAAC-3' (SEQ ID NO: 4)),
500 nM Reverse primer (5'-acggggcgcacctctctttacgcgg-3' (SEQ ID NO: 5)),
300 nM Probe (5'FAM-CCGTGTGCACTTCGCTTCACCTCTGC-3'TAMRA (SEQ ID NO: 6))
were used, and the Plasmid-Safe ATP-dependent DNase-treated product (equivalent to 50 ng total DNA) was measured according to the protocol of THUNDERBIRD Probe qPCR Mix (TOYOBO).

Real-time PCR of rcDNA was performed using a CFX95 Real-time System (Bio-Rad) for 54 cycles of 50°C for 2 minutes, 95°C for 1 minute, and then 95°C for 20 seconds and 60°C for 1 minute. Real-time PCR of cccDNA was performed using a CFX95 Real-time System (Bio-Rad) for 54 cycles of 50°C for 2 minutes, 95°C for 5 minutes, and then 95°C for 20 seconds and 65°C for 1 minute.

### Result:

rcDNA and cccDNA levels were reduced in a manner dependent on the amount of the IFNλ1 protein.

### (6) Figure 8

### Method:

From the livers of human liver chimeric mice persistently infected with HBV which had been administered with vehicle or GalNAc-LNP/pIC (0.01 mg/kg/day), RNA was extracted and provided for microarray analysis.

The frozen livers were each homogenized in 1 mL of a guanidium thiocyanate solution (6 M guanidium thiocyanate, 37.5 mM sodium citrate, 0.75% lauroylsarcosine sodium salt, 1.4% 2-mercaptoethanol).

After addition of 27 µL of 3 M sodium acetate, the homogenate was mixed with 400 µL of acidic saturated phenol. The homogenate was mixed with 80 µL of chloroform and cooled on ice for 15 minutes, followed by centrifugation (at 4°C at 20000 × g for 20 minutes) to separate an aqueous layer (220 µL) which was then transferred to a new tube.

The aqueous layer was mixed with 22 µL of 3 M sodium acetate and 660 µL of 99% ethanol, and then cooled at -20°C for 2 hours. RNA was collected by centrifugation (at 4°C at 20000 × g for 10 minutes).

The collected RNA was purified again according to the protocol of an RNeasy Mini kit (QIAGEN).

The microarray analysis was performed with an Agilent Whole Human Genome ver. 2.0 (4 × 44k type) microarray.

### Result:

GalNAc-LNP/pIC administration specifically induced the expression of type III IFN mRNAs in the liver (IFNL1 (IL29) 4.1, IFNL2 (IL28A) 9.9, IFNL3 (IL28B) 15.7). It should be noted that "IFNL" is intended to mean a gene encoding "IFNλ" in this example and each drawing.

Moreover, among the members of the IRF family, only the expression of IRF1 mRNA was induced (IRF1 4.9).

### (7) Figure 9

### Method:

HepG2 cells made susceptible to HBV infection (HepG2-hNTCP-30 cells) were transfected with a 1.3-fold longer gene of HBV genotype C to prepare cells stably expressing HBV

The primary human hepatocyte-derived human IRF1 gene (GenBank Accession No. NM_002198.3) was cloned into a pCI-neo Mammalian Expression Vector (Promega) to construct an IRF1 plasmid.

Using Lipofectamine 3000 (Invitrogen), the IRF1 plasmid (0.5 µg/well) was introduced by reverse transfection into HepG2 cells stably expressing HBV (2 × 10⁵ cells/well, 48-well plate).

As an empty plasmid, a pCI-neo Mammalian Expression Vector was used.

Using the culture supernatant of the HepG2 cells stably expressing HBV (2 × 10⁵ cells/well, 48-well plate) transfected with the IRF1 plasmid or the empty plasmid (0.5 µg/well), untransfected HepG2 cells stably expressing HBV (2 × 10⁵ cells/well, 48-well plate) were treated every day for 5 days. Likewise, HepG2 cells stably expressing HBV were treated for 5 days with a medium containing recombinant human IFN-lambda 1 (100 ng/mL, R&D systems).

DNA in the cells were extracted by the sodium iodide method.

The cells were mixed with 200 µL of lysis buffer (10 mM EDTA, 500 mM 2-mercaptoethanol, 50 mM Tris-HCl, 75 mM KCl, 5% sarcosyl, 0.1 µg/µL protease K, pH 8.0), followed by heating at 55°C for 20 minutes.

After addition of 300 µL of a NaI solution (40 mM Tris-HCl, 20 mM EDTA, 7.6 M sodium iodide, coprecipitant), the mixture was allowed to stand at room temperature for 15 minutes. After addition of 600 µL of mixed alcohols (40% isopropanol, 60% 1-butanol), the mixture was cooled on ice for 15 minutes. The mixture was centrifuged at 4°C at 15,000 × g for 20 minutes to precipitate DNA. The DNA was washed twice with 70% ethanol and dissolved in TE buffer.

For measurement of cccDNA,
300 nM Forward primer (5'-CAAGGCACAGCTTGGAGGCTTGAAC-3' (SEQ ID NO: 4)),
500 nM Reverse primer (5'-acggggcgcacctctctttacgcgg-3' (SEQ ID NO: 5)),
300 nM Probe (5'FAM-CCGTGTGCACTTCGCTTCACCTCTGC-3'TAMRA (SEQ ID NO: 6))
were used, and the Plasmid-Safe ATP-dependent DNase-treated product (equivalent to 50 ng total DNA) was measured according to the protocol of THUNDERBIRD Probe qPCR Mix (TOYOBO).

Real-time PCR of cccDNA was performed using a CFX95 Real-time System (Bio-Rad) for 54 cycles of 50°C for 2 minutes, 95°C for 5 minutes, and then 95°C for 20 seconds and 65°C for 1 minute.

### Result:

In the IRF1-expressing cells, a significant reduction in cccDNA was observed at 5 days. On the other hand, cccDNA was not reduced during this period in the culture supernatant of the IRF1-expressing cells and in the IFNλ1-treated cells.

### (8) Figure 10

### Method:

From the livers of human liver chimeric mice persistently infected with HBV which had been administered with vehicle or GalNAc-LNP/pIC (0.01 mg/kg/day), RNA was extracted and provided for microarray analysis.

The frozen livers were each homogenized in 1 mL of a guanidium thiocyanate solution (6 M guanidium thiocyanate, 37.5 mM sodium citrate, 0.75% lauroylsarcosine sodium salt, 1.4% 2-mercaptoethanol).

After addition of 27 µL of 3 M sodium acetate, the homogenate was mixed with 400 µL of acidic saturated phenol. The homogenate was mixed with 80 µL of chloroform and cooled on ice for 15 minutes, followed by centrifugation (at 4°C at 20000 × g for 20 minutes) to separate an aqueous layer (220 µL) which was then transferred to a new tube. The aqueous layer was mixed with 22 µL of 3 M sodium acetate and 660 µL of 99% ethanol, and then cooled at -20°C for 2 hours. RNA was collected by centrifugation (at 4°C at 20000 × g for 10 minutes).

The collected RNA was purified again according to the protocol of an RNeasy Mini kit (QIAGEN).

The microarray analysis was performed with an Agilent Whole Human Genome ver. 2.0 (4 × 44k type) microarray.

The expression of Dock11 mRNA reported to be a factor important for maintenance of cccDNA was compared.

### Result:

Dock11 mRNA in the hepatocytes was reduced upon GalNAc-LNP/pIC administration.

### (9) Figure 11

### Method:

HepG2 cells stably expressing HBV (HepG2.2.15 cells) were transfected with the IRF1 expression plasmid or the empty plasmid, and Dock11 mRNA after 48 hours was quantified.

The IRF1 plasmid (0.5 µg/well) or the pCl-neo plasmid was introduced by reverse transfection into HepG2.2.15 cells (2 × 10⁵ cells/well, 48-well plate) using Lipofectamine 3000 (Invitrogen), and the cells were collected after 48 hours.

RNA was extracted according to the protocol of ReliaPrep RNA Miniprep Systems (Promega).

For measurement of human Dock 11,
300 nM Forward primer (5'-TGGAGATTTTTCAGAGGCTGC-3' (SEQ ID NO: 7)) and
300 nM Reverse primer (5'-AACTCCAGCAAGACCTCTTCA-3' (SEQ ID NO: 8))
were added to 100 ng RNA, followed by measurement according to the protocol of RNA-direct SYBR Green Realtime PCR Master Mix (TOYOBO).

Real-time RT-PCR was performed using a CFX95 Real-time System (Bio-Rad) for 45 cycles of 90°C for 30 seconds, 61°C for 20 minutes, 95°C for 1 minute, and then 95°C for 15 seconds and 74°C for 30 seconds.

Human actin beta mRNA was measured as an endogenous control using the following primers to normalize the measured values of Dock11:
300 nM Forward primer (5'-GCACAGAGCCTCGCCTT-3' (SEQ ID NO: 9)) and
300 nM Reverse primer (5'-CCTTGCACATGCCGGAG-3' (SEQ ID NO: 10)).

### Result:

IRF1 expression was found to reduce the expression of Dock11 mRNA.

### (10) Figure 12A

### Method:

The hIFNL1 promoter (-2015 to +186 bp) and the hDock11 promoter (-2022 to +130 bp) were each amplified with Q5 High-Fidelity DNA Polymerase (New England Biolabs) by using primary human hepatocyte gDNA as a template, and then inserted upstream of the Firefly Luciferase gene in the pGL4.51 vector (Promega) in such a way as to form a fusion protein with Firefly Luciferase.

The N-terminal 57 amino acids of IFNλ1 protein or the N-terminal 34 amino acids of Dock11 protein are linked in frame to the N-terminal end of Luciferase protein.

A series of mutant vectors were constructed to lack three putative IRF1-binding sequences (-1285, -1121 and -453 bp) (TTTC or GAAA) in the Dock11 promoter.

### (11) Figure 12B

### Method:

HuH7 cells (1.6 × 10⁶ cells/well) were seeded in 48-well plates.

On the following day, the promoter/Luciferase vectors (25 ng/well) were each transfected together with phRL-TK(Int-) (10 ng/well, Promega) using the IRF1 plasmid (250 ng/well) or the empty plasmid and Lipofectamine 3000 (Invitrogen).

The cells after 48 hours were washed with PBS, and lysed with 70 µL of Cell Culture Lysis Reagent (Promega).

According to the protocols of a Luciferase Assay System and a Renilla Luciferase Assay System (Promega), Firefly and Renilla Luciferase activities were measured with an EnVision reader (PerkinElmer).

The value of Firefly Luciferase activity was normalized with the value of Renilla Luciferase activity.

The Luciferase activity ratio of the IRF1 plasmid-transfected cells to the empty plasmid-transfected cells was shown in graphical form.

### Result:

IRF1 was found to activate the IFNL1 promoter. On the other hand, IRF1 was found to inhibit the activity of the Dock11 promoter. From the experiments using the plasmids lacking IRF1-binding sequences, it was suggested that IRF 1 would bind to and thereby negatively regulate the Dock11 promoter.

### <Discussion>

GalNAc-LNP/pIC was shown to be a natural immunity inducer effectively inducing IFNλ in human liver chimeric mouse hepatocytes without eliciting hepatotoxicity. Moreover, GalNAc-LNP/pIC was deemed to enhance the expression of IRF1 and to effectively reduce HBV cccDNA levels by a plurality of pathways including IFNλ induction. Thus, GalNAc-LNP/pIC according to the therapeutic agent of the present invention was demonstrated to be useful as a therapeutic agent for refractory viral infections, particularly as a therapeutic agent for hepatitis B. GalNAc-LNP/pIC was also demonstrated to be useful as an IFN-λ inducer, an IRF1 expression enhancer, and a Dock11 expression inhibitor, particularly in hepatocytes.

### [Example 2]

"GalNAc-LNP/pIC" prepared in Example 1 and "entecavir" (common name: entecavir hydrate; Bristol Myers Squibb), which is one of the reverse transcriptase inhibitors for the treatment of chronic hepatitis B, were combined for use and confirmed to exert a reducing effect on HBV rcDNA and cccDNA.

### <Method>

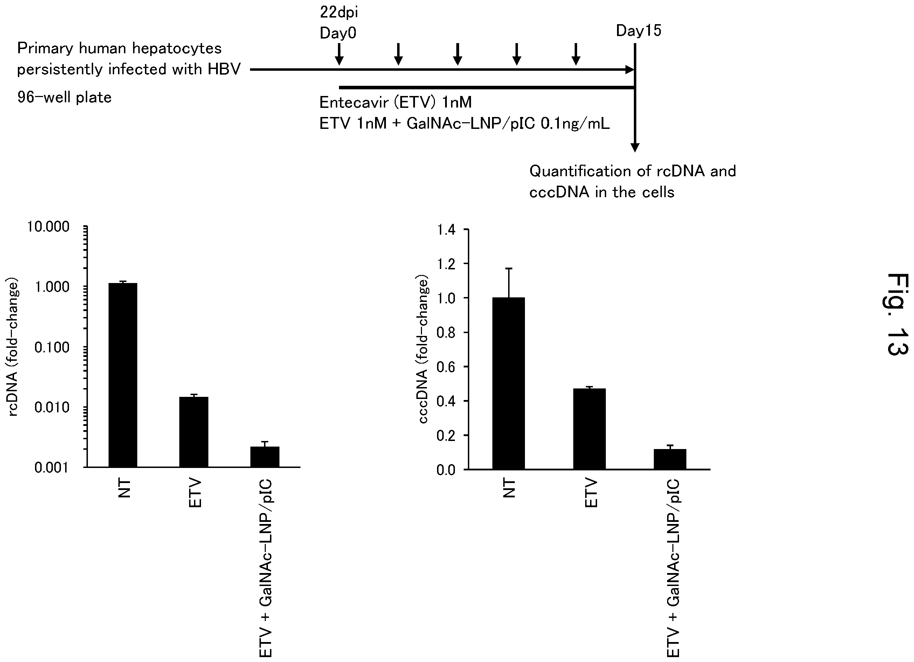

Primary human hepatocytes persistently infected with HBV were treated for 15 days with 1 nM entecavir (ETV) alone (monotherapy) or with 1 nM entecavir (ETV) and 0.1 ng/mL GalNAc-LNP/pIC while conducing medium replacement every 3 days. The cells were collected after 15 days, and rcDNA and cccDNA were quantified (see the upper panel of Figure 13).

DNA in the cells were extracted by the sodium iodide method.

The cells were mixed with 200 µL of lysis buffer (10 mM EDTA, 500 mM 2-mercaptoethanol, 50 mM Tris-HCl, 75 mM KCl, 5% sarcosyl, 0.1 µg/µL protease K, pH 8.0), followed by heating at 55°C for 20 minutes. After addition of 300 µL of a NaI solution (40 mM Tris-HCl, 20 mM EDTA, 7.6 M sodium iodide, coprecipitant), the mixture was allowed to stand at room temperature for 15 minutes. After addition of 600 µL of mixed alcohols (40% isopropanol, 60% 1-butanol), the mixture was cooled on ice for 15 minutes. The mixture was centrifuged at 4°C at 15,000 × g for 20 minutes to precipitate DNA. The DNA was washed twice with 70% ethanol and dissolved in TE buffer.

For measurement of rcDNA, the following primers and probe targeting HBV:
200 nM Forward primer (5'-CACATCAGGATTCCTAGGACC-3' (SEQ ID NO: 1)),
200 nM Reverse primer (5'-AGGTTGGTGAGTGATTGGAG-3' (SEQ ID NO: 2)),
300 nM Probe (5'FAM-CAGAGTCTAGACTCGTGGTGGACTTC-3'TAMRA (SEQ ID NO: 3))
were used, and an aliquot equivalent to 250 ng gDNA was measured according to the protocol of THUNDERBIRD Probe qPCR Mix (TOYOBO).

For measurement of cccDNA,
300 nM Forward primer (5'-CAAGGCACAGCTTGGAGGCTTGAAC-3' (SEQ ID NO: 4)),
500 nM Reverse primer (5'-acggggcgcacctctctttacgcgg-3' (SEQ ID NO: 5)),
300 nM Probe (5'FAM-CCGTGTGCACTTCGCTTCACCTCTGC-3'TAMRA (SEQ ID NO: 6))
were used, and the Plasmid-Safe ATP-dependent DNase-treated product (equivalent to DNA 50 ng) was quantified using THUNDERBIRD Probe qPCR Mix (TOYOBO).

Real-time PCR of rcDNA was performed using a CFX95 Real-time System (Bio-Rad) for 54 cycles of 50°C for 2 minutes, 95°C for 1 minute, and then 95°C for 20 seconds and 60°C for 1 minute. Real-time PCR of cccDNA was performed using a CFX95 Real-time System (Bio-Rad) for 54 cycles of 50°C for 2 minutes, 95°C for 5 minutes, and then 95°C for 20 seconds and 65°C for 1 minute.

### <Result>

As shown in Figure 13 (lower two graphs), rcDNA and cccDNA levels were effectively reduced by the combined use of 1 nM entecavir (ETV) and GalNAc-LNP/pIC when compared to entecavir alone. The reducing effect induced by the combined use was particularly significant on cccDNA levels.

### Industrial Applicability

According to the present invention, it is possible to provide a therapeutic agent for refractory viral infections, particularly hepatitis virus infections (especially hepatitis B virus infection), a pharmaceutical composition for treating the same, and a method for treating the same. The therapeutic agent and others are extremely useful because they can be aimed at complete cure of refractory viral infections in terms of being capable of reducing or eliminating viral cccDNA from hepatocytes (hepatic parenchymal cells). Further, according to the present invention, it is also possible to provide an IFNλ inducer, an IRF1 expression enhancer, a Dock11 expression inhibitor and so on, which can be expected to reduce or eliminate the cccDNA. Moreover, according to the present invention, it is also possible to provide an agent for reducing refractory viral DNA or cccDNA levels and a method for reducing refractory viral DNA or cccDNA levels.

### Sequence Listing Free Text

SEQ ID NO: 1: synthetic DNA
SEQ ID NO: 2: synthetic DNA
SEQ ID NO: 3: synthetic DNA
SEQ ID NO: 4: synthetic DNA
SEQ ID NO: 5: synthetic DNA
SEQ ID NO: 6: synthetic DNA
SEQ ID NO: 7: synthetic DNA
SEQ ID NO: 8: synthetic DNA
SEQ ID NO: 9: synthetic DNA
SEQ ID NO: 10: synthetic DNA

## Claims

1. A therapeutic agent for refractory viral infections, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.

2. The therapeutic agent according to claim 1, wherein the molecule imparting the ability to accumulate in hepatocytes is at least one selected from the group consisting of N-acetylgalactosamine, galactose and lactose.

3. The therapeutic agent according to claim 1 or 2, wherein the drug carrier is at least one selected from the group consisting of lipid nanoparticles, cationic liposomes, atelocollagen and polymeric micelles.

4. The therapeutic agent according to any one of claims 1 to 3, wherein the poly-I, poly-I analog, poly-C and poly-C analog each independently have a chain length in the range of 50 to 1000 bases.

5. The therapeutic agent according to any one of claims 1 to 4, wherein the refractory virus is a hepatitis virus.

6. The therapeutic agent according to claim 5, wherein the hepatitis virus is hepatitis B virus.

7. The therapeutic agent according to any one of claims 1 to 6, which further comprises a pharmaceutically acceptable additional therapeutic agent for viral infections or is used in combination with the additional therapeutic agent for viral infections, wherein the additional therapeutic agent for viral infections is preferably a nucleos(t)ide analogue.

8. A pharmaceutical composition for treating refractory viral infections, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.

9. The pharmaceutical composition according to claim 8, which further comprises a pharmaceutically acceptable additional therapeutic agent for viral infections or is used in combination with the additional therapeutic agent for viral infections, wherein the additional therapeutic agent for viral infections is preferably a nucleos(t)ide analogue.

10. A method for treating refractory viral infections, which comprises administering the therapeutic agent according to any one of claims 1 to 7 or the pharmaceutical composition according to claim 8 or 9 to patients with refractory viral infections.

11. An IFNλ inducer, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.

12. The inducer according to claim 11, which induces IFNλ in hepatocytes.

13. A method for inducing IFNλ, which comprises the step of allowing the inducer according to claim 11 or 12 to act on hepatocytes.

14. An IRF1 expression enhancer, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.

15. The enhancer according to claim 14, which enhances IRF1 expression in hepatocytes.

16. A method for enhancing IRF1 expression, which comprises the step of allowing the enhancer according to claim 14 or 15 to act on hepatocytes.

17. A Dock11 expression inhibitor, which comprises a complex encapsulating poly-I or a poly-I analog and poly-C or a poly-C analog within a drug carrier useful for drug delivery into cells, wherein the surface of the carrier is conjugated with a molecule imparting the ability to accumulate in hepatocytes.

18. The inhibitor according to claim 17, which inhibits Dock11 expression in hepatocytes.

19. A method for inhibiting Dock11 expression, which comprises the step of allowing the inhibitor according to claim 17 or 18 to act on hepatocytes.

20. A therapeutic agent for refractory viral infections, which comprises IFNλ and/or IRF1.

21. A pharmaceutical composition for treating refractory viral infections, which comprises IFNλ and/or IRF1.

22. An agent for reducing refractory viral DNA or cccDNA levels, which comprises IFNλ and/or IRF1.

23. A method for reducing refractory viral DNA or cccDNA levels, which comprises allowing IFNλ and/or IRF1 to act on hepatocytes or allowing IFNλ and/or IRF1 to be expressed in hepatocytes.


SEQUENCE LISTING

<110>  TOKYO METROPOLITAN INSTITUTE OF MEDICAL SCIENCE
       KUMAMOTO UNIVERSITY

<120>  Therapeutic agent for chronic viral infections

<130>  G3003WO

<150>  JP 2021-183329
<151>  2021-11-10

<160>  10

<170>  PatentIn version 3.5

<210>  1
<211>  21
<212>  DNA
<213>  Artificial

<220>
<223>  Synthetic DNA

<400>  1
cacatcagga ttcctaggac c                                                21


<210>  2
<211>  20
<212>  DNA
<213>  Artificial

<220>
<223>  Synthetic DNA

<400>  2
aggttggtga gtgattggag                                                  20


<210>  3
<211>  26
<212>  DNA
<213>  Artificial

<220>
<223>  Synthetic DNA

<400>  3
cagagtctag actcgtggtg gacttc                                           26


<210>  4
<211>  25
<212>  DNA
<213>  Artificial

<220>

<223>  Synthetic DNA

<400>  4
caaggcacag cttggaggct tgaac                                          25


<210>  5
<211>  25
<212>  DNA
<213>  Artificial

<220>
<223>  Synthetic DNA

<400>  5
acggggcgca cctctcttta cgcgg                                          25


<210>  6
<211>  26
<212>  DNA
<213>  Artificial

<220>
<223>  Synthetic DNA

<400>  6
ccgtgtgcac ttcgcttcac ctctgc                                         26


<210>  7
<211>  21
<212>  DNA
<213>  Artificial

<220>
<223>  Synthetic DNA

<400>  7
tggagatttt tcagaggctg c                                              21


<210>  8
<211>  21
<212>  DNA
<213>  Artificial

<220>
<223>  Synthetic DNA

<400>  8
aactccagca agacctcttc a                                              21


<210>  9
<211>  17
<212>  DNA
<213>  Artificial

<220>
<223>  Synthetic DNA

<400>  9
gcacagagcc tcgcctt                                                    17


<210>  10
<211>  17
<212>  DNA
<213>  Artificial

<220>
<223>  Synthetic DNA

<400>  10
ccttgcacat gccggag                                                    17